(19) Europäisches Patentamt / European Patent Office / Office européen des brevets

(11) **EP 2 947 387 B1**

(12) **EUROPÄISCHE PATENTSCHRIFT**

(45) Veröffentlichungstag und Bekanntmachung des Hinweises auf die Patenterteilung:
**21.03.2018   Patentblatt 2018/12**

(51) Int Cl.:
*F23G 7/06* (2006.01)     *F23B 99/00* (2006.01)
*B01D 53/62* (2006.01)     *C01B 21/06* (2006.01)
*C01C 1/02* (2006.01)     *C07C 29/151* (2006.01)

(21) Anmeldenummer: **15001258.1**

(22) Anmeldetag: **16.09.2011**

(54) **STOFFVERWERTUNG  DER STOFFE KOHLENSTOFFDIOXID UND STICKSTOFF  MIT ELEKTROPOSITIVEM METALL**

MATERIAL UTILISATION OF CARBON DIOXIDE AND NITROGEN WITH ELECTROPOSITIVE METAL

RECYCLAGE DE GAZ CARBONIQUE ET D'AZOTE AVEC DU MÉTAL ÉLECTROPOSITIF

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.09.2010   DE 102010041033**

(43) Veröffentlichungstag der Anmeldung:
**25.11.2015   Patentblatt 2015/48**

(62) Dokumentnummer(n) der früheren Anmeldung(en) nach Art. 76 EPÜ:
**14003134.5 / 2 816 284**
**11760755.6 / 2 603 737**

(73) Patentinhaber: **Siemens Aktiengesellschaft**
**80333 München (DE)**

(72) Erfinder: **Schmid, Günter**
**91334 Hemhofen (DE)**

(56) Entgegenhaltungen:
**DE-A1- 4 334 257        DE-A1-102008 031 437**
**DE-A1-102009 014 026    US-A- 2 138 122**

• **Ausfelder ET AL: "Verwertung und Speicherung von CO2", , 31. Oktober 2008 (2008-10-31), Seiten 1-35, XP055042702, Gefunden im Internet: URL:http://www.dechema.de/dechema_media/diskussionco2.pdf [gefunden am 2012-10-30]**

EP 2 947 387 B1

**Beschreibung**

Die vorliegende Erfindung betrifft die industrielle Stoffverwertung von Stoffen, die insbesondere im Kraftwerksbetrieb als Abfallprodukte anfallen. Die Erfindung eignet sich besonders aber nicht ausschließlich zur Verarbeitung von Kohlendioxid oder Stickstoff.
Im Bereich der Kraftwerkskonzepte ist das Verfahren bekannt, in dem Kohle mit reinem Sauerstoff und zurückgeführtem Rauchgas verbrannt wird, im Unterschied zur herkömmlichen Verbrennung von Kohle mit Luft. Dazu wird vor dem Verbrennungsschritt Stickstoff und Sauerstoff aus der Luft abgetrennt. Es wird dadurch eine Anreicherung bzw. Aufkonzentrierung des $CO_2$ im Rauchgas erreicht. Diesen Prozess findet man im Stand der Technik unter dem Begriff Oxyfuel-Prozess. Das bei der Verbrennung entstehende $CO_2$ kann anschließend hochkonzentriert aufgefangen werden. Bei entsprechenden bekannten Oxyfuel-Prozessen fällt das $CO_2$ so hochdicht an, dass es anschließend sequestriert werden kann, d.h. beispielsweise in unterirdischen Kammern gespeichert. Der Oxyfuel-Prozess wird oftmals als "emissionsfrei" bezeichnet. Emissionsfrei in diesem Zusammenhang bedeutet nur dass das $CO_2$ nicht in die Atmosphäre entlassen wird. Es wird gespeichert, aber nicht vermieden. Dokument DE102009014026 A1 offenbart ein Verfahren und eine Vorrichtung zur Stoffverwertung von Kohlendioxid durch Verbrennung mit Magnesium. Es ist Aufgabe der vorliegenden Erfindung, ein Verfahren sowie eine Vorrichtung anzugeben, womit Stoffe, insbesondere Abfallprodukte, verwertet und weiterverarbeitet werden können. Insbesondere soll die $CO_2$-Emission reduziert und eine $CO_2$-Sequestration vermieden werden. Die Aufgabe ist durch ein Verfahren gemäß dem Patentanspruch 1 gelöst. Eine zugehörige Vorrichtung ist im Patentanspruch 7 angegeben. Vorteilhafte Ausgestaltungen der Vorrichtung und Weiterbildungen des Verfahrens sind Gegenstand der Unteransprüche.

Das erfindungsgemäße Verfahren zur industriellen Stoffverwertung umfasst einen Verbrennungsschritt und einen Reaktionsschritt. In dem Reaktionsschritt findet die Umsetzung wenigstens eines Verbrennungsproduktes des Verbrennungsschrittes statt. Der Verbrennungsschritt umfasst eine Reaktion eines elektropositiven Metalls mit dem zu verwertenden Stoff, insbesondere eine exotherme Reaktion. In dem Reaktionsschritt wird zumindest ein Verbrennungsprodukt des Verbrennungsschrittes weiter umgesetzt. Der Reaktionsschritt kann direkt auf den Verbrennungsschritt folgen. Alternativ können die Verbrennungsprodukte aufgefangen und/oder gelagert und/oder transportiert werden, bevor sie dem Reaktionsschritt zugeführt werden. Verbrennungs- und Reaktionsschritt können also örtlich und zeitlich unabhängig voneinander sein.

Mit dem Verfahren werden erfindungsgemäß die Stoffe Stickstoff und Kohlendioxid verwertet. Der Stoff zur Verwertung kann flüssig oder gasförmig vorliegen. Zweckdienlich ist das Verfahren für die Verwertung von Stickstoff und Kohlendioxid, beziehungsweise Gasgemischen, die Stickstoff und Kohlendioxid, ggf. noch verunreinigt, enthalten.

Das Verfahren zur Stoffverwertung, insbesondere von Stickstoff- und Kohlendioxid, kann unabhängig von einem Prozess zur elektrischen Energieerzeugung eingesetzt werden. Insbesondere findet das Verwertungsverfahren für Stickstoff und Kohlendioxid überall Anwendung, wo Stickstoff und Kohlendioxid in ausreichend hoher Konzentration d.h. insbesondere auch ausreichender Reinheit anfallen. Ein derartiger Kohlendioxid-Verwertungsprozess kann beispielsweise bei bisherigen Kohlendioxid-Speichern angesetzt werden oder bei jeder Verbrennung fossiler Brennstoffe, bei der $CO_2$ niedriger Entropie anfällt. $CO_2$ niedrige Entropie bedeutet hochkonzentriert. Nicht jede beliebige Verbrennungsreaktion mit fossilen Brennstoffen liefert hochkonzentriertes $CO_2$, z.B. nicht wenn die Verbrennung mit Luft stattfindet, da hierbei das $CO_2$ mit dem Stickstoff der Luft verdünnt vorliegen würde.

Das erfindungsgemäße Verfahren zur Stoff-Verwertung umfasst ein Verfahren zur Erzeugung elektrischer Energie, in dem die in dem Verbrennungsschritt erzeugte kraftwerkstechnisch nutzbare thermische Energie in elektrische Energie umgewandelt wird.

In dem erfindungsgemäßen Verfahren wird eine Abtrennung von Sauerstoff und Stickstoff aus der Luft durchgeführt. Des Weiteren wird erfindungsgemäß eine Verbrennung eines Brennstoffes mit dem Sauerstoff durchgeführt. Dabei entsteht Kohlendioxid mit niedriger Entropie, d.h. hochkonzentriertes Kohlendioxid. Der Stickstoff, der aus der Luft abgetrennt wurde, und/oder das entstandene Kohlendioxid werden vorzugsweise dem Verwertungs-Verfahren zugeführt. Dieses umfasst die Verarbeitung des Kohlendioxids und des Stickstoffs oder dessen Mischungen. Die Verwertung erfolgt über eine Reaktion mit einem elektropositiven Metall. Hieraus entsteht der Vorteil, dass das $CO_2$ nahezu vollständig aufgearbeitet wird, und ausschließlich umweltunschädliche bzw. weiter nutzbare Endprodukte übrig bleiben. Das Verfahren hat also den Vorteil, elektrische Energie zu erzeugen und eine nahezu vollständige Verwertung von Neben- und Abfallprodukten z.B. der elektrischen Energieerzeugung zu gewährleisten.

Die Kohlendioxid-Verwertung bietet den Vorteil, ein $CO_2$-emissionsfreies Kraftwerk realisieren zu können. Dazu kann der Prozess der Kohlendioxid-Verwertung an den Prozess zur elektrischen Energieerzeugung durch ein Kraftwerk angeschlossen werden. Das Kohlendioxid, das das Kraftwerk verlässt, kann beispielsweise durch den $CO_2$-Verwertungsprozess in Methanol umgewandelt werden. Ebenso kann der Stickstoff-Verwertungsprozess an einen Prozess zur elektrischen Energieerzeugung, z.B. durch ein Kraftwerk, angeschlossen werden.

Der aus der Luft abgetrennte Stickstoff wird dann z.B. gemäß der Erfindung weiterverarbeitet. Dabei wird z.B. in dem Verbrennungsschritt eine Reaktion des elektropositiven Metalls mit dem Stickstoff so geführt, dass ein Nitrid des elektropositiven Metalls entsteht, und wobei in dem Reaktionsschritt eine Hydrolyse durchgeführt wird, bei der aus dem Nitrid durch Zugabe von Wasser Ammoniak hergestellt wird.

In einer bevorzugten Ausführungsform umfasst die Verwertung des Stickstoffs einen Prozessschritt, bei dem mittels des elektropositiven Metalls der Stickstoff und das Wasser direkt zu Ammoniak umgesetzt werden. In einer weiteren vorteilhaften Ausführungsform umfasst die Verwertung des Stickstoffs einen Prozessschritt, bei dem mittels des elektropositiven Metalls der Stickstoff und das Wasser unmittelbar sequentiell zu Ammoniak umgesetzt werden. Dabei entsteht also z.B. Ammoniak als Endprodukt. Die Ammoniakerzeugung bietet den Vorteil, dass dadurch andere Ammoniakerzeugungsprozesse ersetzt werden können. Ammoniak stellt insbesondere einen der wichtigsten Rohstoffe der Düngemittelindustrie dar. Bisher wird Ammoniak nach dem Haber-Bosch-Prozess hergestellt. Ein erfindungsgemäßes Verfahren zur Verwertung von Stickstoff hat also den Vorteil, eines der wichtigsten Ausgangsstoffe zu erzeugen, das für die heutige Energie und chemische Stoffwirtschaft notwendig ist, da diese auf fossilen Brennstoffen basiert. Vorzugsweise wird der Stickstoff-Verwertungsprozess als Ammoniaksynthese-Prozess eingesetzt. Mit dem erfindungsgemäßen Stickstoff-Verwertungsprozess kann Stickstoff aus der Luft gebunden werden und für die Nahrungs- und Biomasseproduktion zur Verfügung gestellt werden, ohne dass dafür fossile Brennstoffe bereitgestellt werden müssen.

Der Oxyfuel-Prozess ist ein Beispiel für einen Prozess zur Erzeugung elektrischer Energie, bei dem Stickstoff und Kohlendioxid in ausreichender Menge, ausreichender Reinheit sowie geeigneter Konzentration zur Verfügung stehen. Der erfindungsgemäße Verwertungsprozess wird vorzugsweise an den Oxyfuel-Prozess angeschlossen. Durch den erfindungsgemäßen Verwertungsprozess im Sinne eines Post-Oxyfuel-Prozess wird ein emissionsfreies Kraftwerkskonzept geschaffen.

In einer vorteilhaften Ausgestaltung der Erfindung ist das Verfahren so ausgestaltet, dass die Verarbeitung des Kohlendioxids einen Verbrennungsschritt und einen Syntheseschritt umfasst. Dabei umfasst wiederum der Verbrennungsschritt eine exotherme Reaktion des elektropositiven Metalls mit dem Kohlendioxid. Dabei wird insbesondere im Zuge der Verbrennung thermische Energie erzeugt. Die Reaktion wird dabei vorzugsweise so geführt, dass am Ende Kohlenmonoxid entsteht. Der Syntheseschritt wiederum umfasst vorzugsweise die Methanolsynthese aus dem Kohlenmonoxid und Wasserstoff. Die chemische Weiterverarbeitung der Verbrennungsprodukte dient also vorzugsweise der Herstellung wertvoller Grundchemikalien.

Bei dem Verbrennungsschritt des elektropositiven Metalls mit dem Kohlendioxid fallen Oxide und Carbonate des elektropositiven Metalls als Abfallprodukte an. Das Kohlendioxid kann mittels elektropositiver Metalle bis hin zu Kohlenstoff reduziert werden. Der in der Brennkammer gebildete Kohlenstoff kann jedoch im Rahmen eines Boudouard-Gleichgewichts mit weiterem $CO_2$ zu Kohlenmonoxid komproportionieren, so dass am Ende formal mittels dem elektropositiven Metall $CO_2$ zu CO reduziert wird.

Der Syntheseschritt umfasst z.B. eine Reaktion mit Wasserstoff. CO und $H_2$ bilden z.B. ein Synthesegas, das unter anderem für die Methanolherstellung verwendet werden kann. Das hat den Vorteil, dass jedes Kohlenstoffatom des fossilen Energieträgers doppelt verwendet wird. In dem ersten Schritt wird es klassisch zu $CO_2$ verbrannt, wobei die Verbrennung so geführt wird, dass es in hochkonzentrierter Form anfällt, z.B. in einem Oxyfuel-Prozess. In einem zweiten Schritt kann es mittels eines elektropositiven Metalls wieder reduziert werden, und beispielsweise in einen Treibstoff für Kraftfahrzeuge umgewandelt werden.

In einer weiteren vorteilhaften Ausgestaltung der Erfindung umfasst das Verfahren die Verarbeitung von Stickstoff, wobei in einem Verbrennungsschritt mit einer exothermen Reaktion des elektropositiven Metalls mit dem Stickstoff ein Nitrid des elektropositiven Metalls entsteht und wobei in einem Hydrolyseschritt aus dem Nitrid durch Zugabe von Wasser Ammoniak hergestellt wird. Insbesondere kann die Verarbeitung des Stickstoffs auch so erfolgen, dass in einem einzigen Prozessschritt mittels des elektropositiven Metalls der Stickstoff und das Wasser direkt zu Ammoniak umgesetzt werden. Alternativ kann die Verarbeitung des Stickstoffs auch so erfolgen, dass in einem einzigen Prozessschritt mittels des elektropositiven Metalls der Stickstoff und das Wasser unmittelbar hintereinander zu Ammoniak umgesetzt werden. Ein Reaktionsschritt kann z.B. darin bestehen, dass im Verbrennungsschritt Wasserdampf zugesetzt wird, so dass Ammoniak in direkter Reaktion entsteht. Dabei beispielsweise wird das Nitrid nur intermediär gebildet.

Ein Vorteil der Stickstoff-Verwertung liegt in dem Endprodukt Ammoniak. Ammoniak stellt einen der wichtigsten Ausgangsstoffe für die Düngemittelindustrie dar. Besonders hinsichtlich des zukünftigen Bedarfs an Biomasse, ist auch mit einem erhöhten Bedarf an Düngemittel zu rechnen. Dieser Bedarf kann dann mittels des erfindungsgemäßen Stickstoff-Verwertungsprozesses, insbesondere in Kombination mit einem Prozess zur Erzeugung elektrischer Energie, beispielsweise einem Oxyfuel-Prozess, gedeckt werden.

Der erfindungsgemäße Verwertungsprozess kann als Post-Oxyfuel-Prozess Einsatz finden und auf Kohlendioxid sowie auf Stickstoff angewendet werden. Vorteilhafterweise werden Ammoniak und Methanol erzeugt. Es handelt sich also um eine chemische Weiterverarbeitung von Abfallprodukten, die zur Herstellung wertvoller weiterverwertbarer Grundchemikalien genutzt wird.

In einer weiteren vorteilhaften Ausgestaltung der Erfindung wird bei dem Verfahren ein Wärmetransport durchgeführt, der thermische Energie, die insbesondere bei dem Verbrennungsschritt mit einer exothermen Reaktion des elektropositiven Metalls frei wird, einem Schritt zur Erzeugung elektrischer Energie zuführt. Schon der Verwertungsprozess für sich genommen erzeugt in dem Verbrennungsschritt Energie in Form von Hochtemperaturwärme, die für die Erzeugung elektrischer Energie, z.B. über eine Dampfturbine, genutzt werden kann. Vorteilhafterweise wird der Verwertungsprozess an einen Prozess zur Erzeugung elektrischer Energie angeschlossen. Die bei der Verbrennung des elektropositiven Metalls mit Stickstoff oder mit Kohlendioxid entstehende zusätzliche Hochtemperaturwärme wird der Erzeugung elektrischer Energie zusätzlich zugeführt.

In einer weiteren beispielhaften Ausgestaltung der Erfindung umfasst das Verfahren einen Rückgewinnungsprozess. In diesem Rückgewinnungsschritt wird ein Oxid und/oder ein Salz des elektropositiven Metalls wieder in das Metall umgesetzt. Eine derartige Wiederaufarbeitung des umgesetzten elektropositiven Metalls hat den Vorteil, dass der Gesamtkreislauf geschlossen werden kann. Eine derartige Wiederaufarbeitung kann beispielsweise eine elektrochemische Reduktion sein. Dabei können insbesondere Oxide, Hydroxide oder Salze des elektropositiven Metalls wieder in das Metall umgesetzt werden. Allgemein kann eine elektrochemische Reduktion des Metallions $M^{n+}$ zu dem Metall M zurückführen. Dafür ist wiederum elektrische Energie notwendig, die beispielsweise aus fotovoltaischer Energie gewonnen werden kann. Insbesondere kann ein derartiger Wiederaufarbeitungs- bzw. Rückgewinnungsprozess des elektropositiven Metalls als Energiespeicher für aus Fotovoltaik gewonnener Energie angesehen werden. Besonders vorteilhaft ist die Durchführung eines Rückgewinnungsprozesses für elektropositive Metalle, die einer natürlichen Vorkommensbegrenzung unterliegen wie z.B. Lithium.

Das erfindungsgemäße Verfahren ist so ausgestaltet, dass ein elektropositives Metall mit einem Normalpotential kleiner als null Volt verwendet wird. Das erfindungsgemäße Verfahren ist so ausgestaltet, dass in dem Verfahren ein elektropositives Metall der ersten oder der zweiten Hauptgruppe verwendet wird. Insbesondere wird Lithium verwendet. Alternativ können auch elektropositive Metalle wie Natrium, Kalium, Calcium, Strontium, Barium, Magnesium oder Zink eingesetzt werden. Die natürlichen Ressourcen an Natrium und Magnesium beispielweise unterliegen keiner Vorkommensbegrenzung. Zwar ist die weltweit vorhandene Menge an Lithium limitiert, jedoch ist mit einer Verknappung erst in ca. 40 Jahren zu rechnen. Hier hat wiederum die Rückgewinnungsmethode den Vorteil den Prozess von einer natürlichen Begrenzung des Lithiums unabhängig zu machen.

Vorteilhafterweise wird für ein Verfahren mit Rückgewinnungsprozess fotovoltaische Energie für die Rückgewinnung des elektropositiven Metalls verwendet. Alternativ ist auch jede andere Form regenerativ gewonnener elektrischer Energie für den elektrochemischen Rückgewinnungsprozess einsetzbar. Der Rückgewinnungsprozess kann von dem Prozess zur Erzeugung elektrischer Energie z.B. dem Oxyfuel-Prozess und den Verwertungsprozessen für Stickstoff und Kohlendioxid z.B. in Form eines Post-Oxyfuel-Prozesses räumlich und zeitlich getrennt stattfinden.

Zur Stoff-Verwertung dient erfindungsgemäß eine Vorrichtung, die eine Brennkammer mit einem elektropositiven Metall umfasst. Dabei dient die Brennkammer zur Verbrennung des elektropositiven Metalls in dem Stoff. Die Brennkammer ist vorzugsweise so ausgestaltet, dass den Stoff, ein Gas oder eine Flüssigkeit in die Brennkammer einleitbar ist und dass ein Verbrennungsschritt mit einer exothermen Reaktion eines elektropositiven Metalls durchführbar ist.

Erfindungsgemäß umfasst die Vorrichtung zur Stoff-Verwertung eine Reaktionskammer, die zur weiteren Umsetzung wenigstens eines Verbrennungsproduktes dient. Dazu ist die Reaktionskammer so ausgestaltet, dass ein Verbrennungsprodukt aus der Brennkammer in die Reaktionskammer einleitbar ist und eine Umsetzung des Verbrennungsproduktes durchführbar ist. Dabei wird aus einer Stickstoff-Verwertung, beispielsweise Ammoniak, und aus einer Kohlendioxid-Verwertung, beispielsweise Methanol, gewonnen.

In einer weiteren vorteilhaften Ausgestaltung der Erfindung umfasst eine Anordnung eine Verwertungs-Vorrichtung und z.B. eine Luftzerlegungsanlage. Mittels der Luftzerlegungsanlage wird z.B. Sauerstoff und Stickstoff aus der Luft abgetrennt.

Erfindungsgemäß umfasst die Verwertungs-Vorrichtung eine Vorrichtung, die zur Erzeugung elektrischer Energie dient. Diese ist erfindungsgemäß ein Kraftwerk. Insbesondere ist eine Dampfturbine umfasst.

Die Vorrichtung umfasst erfindungsgemäß weiter eine Brennkammer, also einen Dampferzeuger, die zur Verbrennung eines Brennstoffes mit dem Sauerstoff ausgestaltet ist. Beispielsweise wird über eine Abscheideeinrichtung das bei der Verbrennung entstandene Kohlendioxid ausgeleitet. Es kann auch eine Abscheideeinrichtung für die Ausleitung des abgetrennten Stickstoffs umfasst sein.

Erfindungsgemäß ist die Verwertungs-Vorrichtung so mit dem Kraftwerk verbunden, dass der abgetrennte Stickstoff in die Brennkammer, bzw. den Dampferzeuger, einleitbar ist. Beispielsweise ist eine zweite Verwertungs-Vorrichtung vorteilhafterweise so mit dem Kraftwerk verbunden, dass das über die Abscheideeinrichtung ausgeleitete Kohlendioxid in die Brennkammer einleitbar ist.

In aktuellen Kraftwerken entsteht als Abfall z.B. ein Gemisch aus Stickstoff und Kohlendioxid. Wird dieses Gemisch mit einem elektropositiven Metall weiterverarbeitet, verläuft die Reaktion mit Kohlendioxid viel heftiger als mit Stickstoff. Insbesondere kann so $CO_2$ als Lithiumcarbonat aus dem Abgas abgetrennt werden.

Der Vorteil der Stickstoff-Verwertung sowie der Kohlendioxid-Verwertung liegt darin, dass bei dem Verbrennungsschritt in der Verbrennungskammer Energie in Form von Hochtemperaturwärme erzeugt wird, die zur Erzeugung elektrischer Energie genutzt werden kann. Vorzugsweise ist eine Vorrichtung zur Stickstoff-Verwertung oder eine Vorrichtung zur Kohlendioxid-Verwertung oder je eine Vorrichtung zur Stickstoff-Verwertung und je eine zur Kohlendioxid-Verwertung an ein Kraftwerk angekoppelt. In dem Fall kann die in dem Verbrennungsschritt in der Brennkammer erzeugte Energie in Form von Hochtemperatur- wärme an das Kraftwerk, insbesondere den Dampferzeuger in dem Kraftwerk zusätzlich zugeführt werden und der Erzeugung elektrischer Energie dienen. Des Weiteren birgt die Kohlendioxid-Verwertung den Vorteil, dass die $CO_2$-Emission verhindert werden kann bzw. stark reduziert werden kann. Das Kohlendioxid, das bei der Verbrennung von fossilen Brennstoffen entsteht, kann mittels der erfindungsgemäßen Vorrichtung nahezu vollständig in ein weiterverwertbares Endprodukt umgewandelt werden. Beispielsweise wird Methanol erzeugt. Ein weiterer Vorteil der Stickstoff-Verwertung liegt daran, dass ein Abfallprodukt wie beispielsweise Ammoniak erzeugt werden kann, was zu einem der wichtigsten Rohstoffe der Düngemittelindustrie zählt. Insgesamt liegt der Vorteil der Erfindung darin, dass die Wertschöpfungskette für den Kraftwerksbauer und Kraftwerksbetreiber verlängert wird.

Die erfindungsgemäße Vorrichtung umfasst eine Verwertungs-Vorrichtung für das Kohlendioxid mit einer Brennkammer und einer Reaktionskammer. Dabei ist die Brennkammer vorzugsweise so ausgestaltet, dass sie für einen Verbrennungsschritt mit einer exothermen Reaktion eines elektropositiven Metalls mit dem Kohlendioxid geeignet ist. Insbesondere ist die Brennkammer so ausgestaltet, dass die Reaktion so führbar ist, dass am Ende Kohlenmonoxid entsteht. Prinzipiell kann bei der exothermen Reaktion des elektropositiven Metalls mit dem Kohlendioxid auch Kohlenstoff erzeugt werden. Dieser kann jedoch über das Boudouard-Gleichgewicht weiter zu Kohlenmonoxid komproportionieren. Die Reaktionskammer ist vorteilhafterweise mit einem Synthesegasreaktor ausgestattet. Insbesondere ist die Reaktionskammer mit einem Synthesegasreaktor gekoppelt, der die Umsetzung von Wasserstoff mit dem Kohlenmonoxid ermöglicht. Beispielsweise wird der Reaktionskammer ein Synthesegasreaktor nachgeschaltet. Der Synthesegasreaktor wiederum kann so ausgestaltet sein, dass er zur Synthese von Methanol aus Kohlenmonoxid und Wasserstoff geeignet ist. Eine derartige Vorrichtung zur Kohlendioxid-Verarbeitung kann beispielsweise überall da angebracht und betrieben werden, wo Kohlendioxid in geeigneter Form anfällt, d.h. mit niedriger Entropie bzw. hochkonzentriert vorliegt. Neben Kraftwerken sind also auch bereits bestehende Kohlendioxidspeicher-Reservoirs als Quelle für die Kohlendioxid-Verwertung denkbar.

Die erfindungsgemäße Vorrichtung umfasst eine Verwertungs-Vorrichtung für den Stickstoff mit einer Brennkammer und einer Reaktionskammer ausgestaltet. Die Brennkammer ist für einen Verbrennungsschritt mit einer exothermen Reaktion eines elektropositiven Metalls mit dem Stickstoff ausgestaltet. Bei einer derartigen Verbrennungsreaktion kann z.B. ein Nitrid des elektropositiven Metalls entstehen oder als Intermediat auftreten. Des Weiteren ist die Reaktionskammer so ausgestaltet, dass darin mittels Hydrolyse von dem Nitrid des elektropositiven Metalls unter Zugabe von Wasser Ammoniak hergestellt werden kann. Alternativ ist eine Hydrolysekammer neben der Reaktionskammer angebracht.

Beispielsweise ist die Erfindung so ausgestaltet, dass die Vorrichtung für die Erzeugung von Ammoniak aus dem Stickstoff eine Verwertungs-Vorrichtung vorsieht, die mit nur einer kombinierten Brenn- und Reaktionskammer ausgestaltet ist. In dieser kombinierten Brenn- und Reaktionskammer ist zweckdienlicherweise eine Prozessführung möglich, so dass mittels des elektropositiven Metalls Stickstoff und Wasser direkt zu Ammoniak umsetzbar sind.

Beispielsweise kann für die Umsetzung bzw. Verwertung von Stickstoff das elektropositive Metall Lithium herangezogen werden. Bei der Verbrennung von Lithium in Stickstoff ergibt sich ein Wert für die Energiefreisetzung von $16 \, \dfrac{kcal}{mol \cdot electron}$. Diese Angabe ist also auf die Anzahl umgesetzter Elektronen bei der Reaktion normiert. Bei der Verbrennung von Kohlenstoff in Sauerstoff ergibt sich ein Wert für die Energiefreisetzung von $24 \, \dfrac{kcal}{mol \cdot electron}$, der also nur gering höher ist als der für die Verbrennung von Lithium in Stickstoff. Das zeigt den ersten Vorteil des Prozesses der Stickstoff-Verwertung, nämlich dass wiederum Energie in Form von Hochtemperaturwärme erzeugt werden kann, die zur Erzeugung elektrischer Energie genutzt werden kann. Das bei dieser Verbrennungsreaktion entstehende Lithiumnitrid muss aber kein Abfallprodukt sein. Erfindungsgemäß kann das Lithiumnitrid weiter umgesetzt werden. Das Lithiumnitrid reagiert z.B. exotherm mit Wasser zu Ammoniak. Ein weiteres Endprodukt dieser Reaktion ist Lithiumhydroxid, was in einer erfindungsgemäßen Rückgewinnungsreaktion wieder zu Lithium reduziert werden kann.

Bisher wird Ammoniak mittels fossiler Brennstoffe hergestellt. Das erfindungsgemäße Stickstoff-Verwertungsverfahren ist ein alternatives Ammoniakherstellungsverfahren. Für die Herstellung von Ammoniak werden 3 % der fossilen Gesamtweltenergie-Aufwände verbraucht. Das zeigt den großen Vorteil der erfindungsgemäßen Stickstoff-Verwertung.

Die Reaktion muss nicht immer sequentiell geführt werden. Beispielsweise kann die Prozessführung in der Brennkammer so gestaltet sein, dass mittels Lithium, Stickstoff und Wasser direkt in Ammoniak umgesetzt werden können. Die Bindung des im Wassermolekül enthaltenen Sauerstoffs kann durch Lithium zu Lithiumoxid erfolgen, was die Wärmetönung der Reaktion insgesamt erhöhen kann.

Des Weiteren kann Lithiumnitrid auch als Superbase mit molekularem Wasserstoff zu Lithiumamid und Lithiumhydrid reagieren. Lithiumhydrid stellt einem potenziellen Hochtemperatur-Wasserstoffspeicher dar, der bei 270°C seinen Wasserstoff wieder reversibel abgibt.

Erfindungsgemäß ist die Verwertungs-Vorrichtung mit einem Kraftwerk (A) zur Erzeugung elektrischer Energie, angeordnet. Zur Erzeugung elektrischer Energie aus der thermischen Energie der Verbrennung des elektropositiven Metalls ist vorteilhafterweise eine Dampfturbine umfasst. Es kann auch je eine Dampfturbine in der Verwertungseinrichtung oder in dem Kraftwerk umfasst sein. Eine Anordnung aus Kraftwerk und zwei Verwertungsvorrichtungen für Stickstoff und Kohlendioxid kann bis zu drei Dampferzeuger umfassen.

In einer weiteren vorteilhaften Ausgestaltung der Erfindung umfasst die Anordnung eine Wärmetransporteinrichtung, die das Kraftwerk mit der Brennkammer einer der Verwertungs-Vorrichtungen verbindet. Insbesondere kann eine Wärmetransporteinrichtung umfasst sein, die das Kraftwerk mit beiden Brennkammern der Verwertungs-Vorrichtungen für Stickstoff und Kohlendioxid verbindet. Die Wärmetransporteinrichtung ist so ausgestaltet, dass thermische Energie, die insbesondere durch einen Verbrennungsschritt in einer der Brennkammern frei wird, transportiert und dem Kraftwerk zur Verfügung gestellt werden kann. Insbesondere kann der Wärmetransport zu einer Brennkammer in dem Kraftwerk gelangen. Beispielsweise wird dem Dampferzeuger in dem Kraftwerk zusätzliche Hochtemperaturwärme aus den Brennkammern der Verwertungseinrichtungen für den Stickstoff und das Kohlendioxid zur Erzeugung elektrischer Energie zugeführt. Diese Ausgestaltung hat den Vorteil einer doppelten Nutzung der ursprünglichen Abfallprodukte Stickstoff und Kohlendioxid. Nicht nur wird der $CO_2$-Ausstoß dadurch stark verringert, sondern durch den Verbrennungsprozess zusätzliche Hochtemperaturwärme für die Erzeugung elektrischer Energie zur Verfügung gestellt. Darüber hinaus hat die Vorrichtung zur Kohlendioxid-Verwertung den Vorteil insbesondere Methanol als Ausgangsprodukt zur Verfügung zu stellen. Die Vorrichtung zur Stickstoff-Verwertung birgt gleichermaßen den Vorteil aus der Verbrennungsreaktion zusätzliche Hochtemperaturwärme zur Verfügung zu stellen, neben dem wichtigen Ausgangsprodukt Ammoniak.

Das zentrale Element für die Stickstoff- und Kohlendioxid-Verwertung ist ein elektropositives Metall, insbesondere Lithium. Dieses wird selbst bei dem Prozess nicht verbraucht, sondern ändert nur die Oxidationsstufe. Mittels eines Rückgewinnungsprozesses kann so der Kreislauf geschlossen werden. Für den Rückgewinnungsprozess wird insbesondere Sonnenenergie genutzt. Elektropositive Metalle, die für den Post-Oxyfuel-Prozess geeignet sind, bilden somit auch Sonnenergiespeicher.

Insbesondere für die Märkte der Erzeugung regenerativer Energien ohne inhärente Speichermöglichkeit wie z.B. Energie aus Sonne, Wind, Gezeiten oder Biomasse sind neue Speichermöglichkeiten von großem zukünftigem Interesse. Dabei kann die Rückgewinnung, also das Recycling der elektropositiven Metalle, die in dem Post-Oxyfuel-Prozess Anwendung finden, angewendet werden. Dafür besteht auch keine Konkurrenzsituation zum Transport elektrischer Energie über weite Strecken mittels Kabel. Beispielsweise wird derzeit auch der Energieträger Kohle oder Erdöl aus den Erzeugerländern transportiert, anstelle diesen in den Erzeugerländern vor Ort in Strom zu verwandeln. Mit elektropositiven Metallen als Energiespeicher, beispielsweise für Sonnenenergie, kann die bestehende Energieerzeugungslandschaft klima- und umweltfreundlicher gestaltet werden, aber auch auf lange Sicht ist in den elektropositiven Metallen als Energiespeicher das Potential zu sehen, dass fossile Energieträger gänzlich abgelöst werden können. Dazu wäre auch eine Verbrennung der elektropositiven Metalle in Luft denkbar.

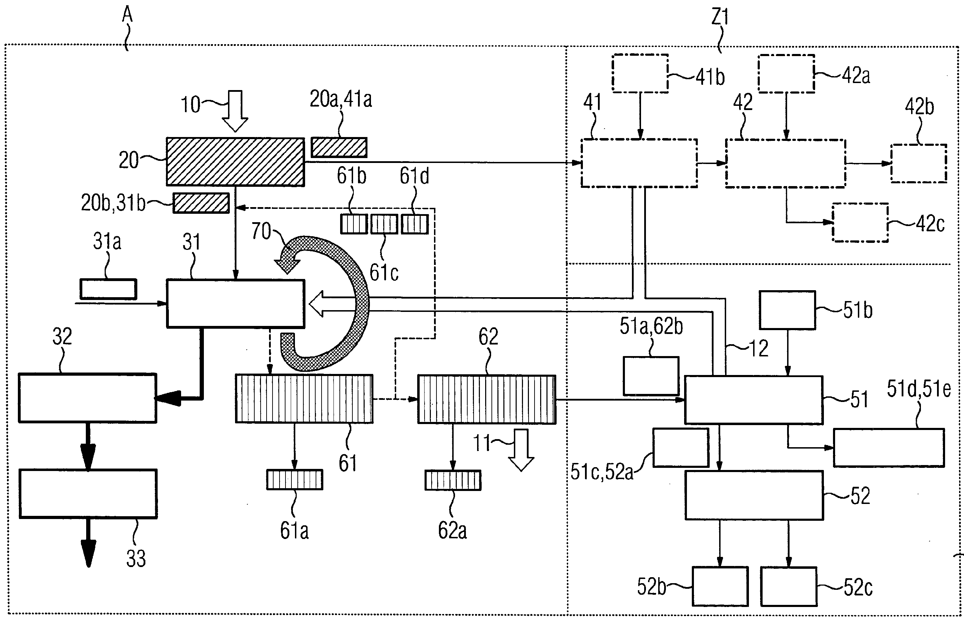

Ausführungsformen der vorliegenden Erfindung werden in exemplarischer Weise mit Bezug auf die Figur beschrieben.

Die Figur zeigt ein Flussdiagramm, das den gesamten Prozessablauf abbildet bzw. die notwendigen Vorrichtungen für ein erfindungsgemäßes Kraftwerk schematisch darstellt. Die zentralen Prozessschritte werden durch große Kästen oder Rechtecke dargestellt, kleine Kästen stellen die Eingangs- und Ausgangsprodukte der Prozessschritte dar. Mit Bezug auf ein Kraftwerk können die Kästen auch exemplarisch für verschiedene Vorrichtungen und Kammern stehen. Die Pfeile zwischen den Kästen deuten den Prozessablauf an, insbesondere den zeitlichen Prozessablauf. In Bezug auf Eingangs- und Ausgangsprodukte der unterschiedlichen Prozessschritte deuten die Pfeile auch das Ein- und Ausleiten der Produkte an. Für das Ein-, Aus- und Weiterleiten von Produkten bzw. des Stoffes stehen einfache Pfeile. Die hohl dargestellten dicken Pfeile stehen für einen Energieeingang, -ausgang oder -transport.

Auf der linken Seite der Figur ist ein Standard-Oxyfuel-Kraftwerk A schematisch dargestellt. Von oben nach unten sind folgende Prozessschritte bzw. Vorrichtungen verbildlicht: Die Luftzerlegungsanlage 20 benötigt zur Zerlegung der Luft einen gewissen Energieeintrag 10. Aus der Luftzerlegungsanlage tritt zu einer Seite Stickstoff 20a aus. Des Weiteren tritt Sauerstoff 20b aus. Der Stickstoff 20a wird in der ersten Weiterverarbeitungsvorrichtung Z1 weiter verarbeitet. Dazu wird er aus dem Kraftwerk A, von der Luftzerlegungsanlage 20 ausgeleitet und stellt ein erstes Eingangsprodukt 41a für die Weiterverwertungs-Vorrichtung Z1 dar. Der Sauerstoff 20b, der aus der Luftzerlegungsanlage 20 austritt wird als Eingangsprodukt 31b in den Dampferzeuger 31 eingeleitet. Des Weiteren werden in den Dampferzeuger 31 Kohle oder allgemein ein fossiler Brennstoff 31a eingeleitet sowie weiterer Sauerstoff 61b, Kohlendioxid 61c und Wasser 61d, das mittels eines Rauchgasrückführungsprozesses 70 wieder in den Dampferzeuger 31 eingeleitet wird. In dem Dampferzeuger 31 findet die Verbrennung des fossilen Brennstoffs 31a, insbesondere der Kohle unter Sauerstoffatmosphäre statt. Mittels darüber erzeugter Wärmeenergie wird eine Dampfturbine 32 und darüber wiederum ein Generator 33 angetrieben, mittels dem elektrische Energie erzeugt werden kann. Alternativ ist es möglich die heißen Gase der Verbrennung direkt auf eine Gasturbine zu leiten.

Die Verbrennungsprodukte gelangen zunächst nach der Verbrennung in dem Dampferzeuger 31 in die Staubabscheidevorrichtung 61. Über diese Staubabscheideeinrichtung 61 wird Staub 61a aus dem Kraftwerk ausgeleitet. Des Weiteren wird Kohlendioxid 61c, Sauerstoff 61b und in Teilen Wasser 61d rezirkuliert, d.h. in die Brennkammer des Dampferzeuger 31 zurückgeführt. Somit wird der noch nicht verbrauchte Sauerstoff 61b für die Verbrennung ein weiteres Mal genutzt und gewährleistet, dass am Ende hochkonzentriertes Kohlendioxid als Ausgangsprodukt zur Verfügung steht, was nicht mehr zum Dampferzeuger zurückgeführt wird. Über die Rauchgas-Reinigungsvorrichtung 62 werden die Ausgangsprodukte Wasser 62a und hochkonzentriertes Kohlendioxid 62b ausgeleitet. Des Weiteren wird von der Rauchgas-Reinigungsvorrichtung 62 Niedertemperaturwärme 11 abgeführt. Die Staubabscheidung 61 und die Rauchgasreinigung 62 bilden zusammen die Abscheideeinrichtung. Das Ausgangsprodukt Kohlendioxid 62b aus der Abscheidevorrichtung wird zur weiteren Verarbeitung in die zweite Verwertungs-Vorrichtung für das Kohlendioxid Z2 eingeleitet. Dort bildet das Kohlendioxid 51a ein erstes Eingangsprodukt in die Brennkammer 51 der Weiterverarbeitungseinrichtung Z2.

Die Weiterverarbeitungseinrichtungen Z1/Z2 sind direkt an das Oxyfuel-Kraftwerk A angebracht, so dass Stickstoff 20a/41a und Kohlendioxid 51a/62b direkt aus dem Kraftwerk ausgeleitet und in die Weiterverarbeitungseinrichtungen Z1/Z2 eingeleitet werden können. Genauer werden der Stickstoff 20a/41a und das Kohlendioxid 51a/62b direkt in die jeweiligen Brennkammern 41/51 der Weiterverarbeitungseinrichtungen Z1/Z2 eingeleitet.

Das Kohlendioxid 41a wird zusammen mit Lithium 41b in der Brennkammer 41 verbrannt. Dabei entsteht Hochtemperatur-Wärme 12, die zusätzlich zur entstandenen Wärme im Kraftwerk zur Dampferzeugung 31 zurückgeführt und genutzt werden kann. Ausgangsprodukt der Brennkammer 41 ist Lithiumnitrid, was in die Hydrolysekammer 42 weitergeleitet wird. Das entstandene Lithiumnitrid ist bei weitem kein Abfallprodukt. Es reagiert exotherm mit Wasser zu Ammoniak 42b. Ammoniak ist eines der wichtigsten Ausgangsprodukte der Düngemittelherstellung. Im jetzigen Stand der Technik wird Ammoniak mittels fossiler Brennstoffe hergestellt. Dabei entfallen 3 % der fossilen Gesamtwelt-Energieaufwände auf die Herstellung von Ammoniak. Ohne Düngemittel ist nämlich keine wirtschaftliche Herstellung von Biomasse möglich.

Beispielsweise ist die Brennkammer so ausgestaltet, dass im oberen bzw. vorderen Teil $Li_3N$ erzeugt wird. Dann fliegt der Staub mit hoher Geschwindigkeit entlang einem Rohr. Im unteren bzw. hinteren Teil der Brennkammer wird dann Wasserdampf eingeleitet. Am Ende wird ein Gas-Staub-Gemisch aus $Li_2O$, Ammoniak und überschüssigem $H_2O$ erhalten.

Normiert man die Verbrennung auf die Zahl umgesetzter Elektronen so ergibt sich bei der Verbrennung von Kohlenstoff in Sauerstoff eine Energiefreisetzung

von $24 \dfrac{kcal}{mol \cdot electron}$. Bei der Verbrennung von Lithium in Stickstoff ergibt sich eine Energiefreisetzung

von $16 \dfrac{kcal}{mol \cdot electron}$. Dieser Wert ist nur etwa 30 % geringer, was die Nutzung der Lithiumverbrennung in Stickstoff kraftwerkstauglich und großtechnisch nutzbar macht.

Aus der Hydrolysekammer 42 treten die Ausgangsprodukte Ammoniak 42b und Lithiumhydroxid 42c aus. Das Lithiumhydroxid kann mittels elektrochemischer Reduktion zu Lithium zurückgeführt werden. Ein benötigtes Eingangsprodukt für den Hydrolyseschritt ist Wasser 42a.

Entsprechend der Weiterverarbeitung von Stickstoff wird auch das Kohlendioxid 62b/51a zur Weiterverarbeitung in die Brennkammer 51 der Weiterverarbeitungseinrichtung, bzw. Verwertungs-Vorrichtung Z2 eingeleitet. Des Weiteren wird in die Brennkammer 51 Lithium 51b eingeleitet. Das Lithium 51b wird unter Kohlendioxidatmosphäre verbrannt. Dabei entsteht wiederum Hochtemperaturwärme 12, die dem Kraftwerk zugeführt werden kann, insbesondere dem Dampferzeugungsprozess 31. Die Reaktion von Lithium mit $CO_2$ ist stark exotherm. Als Ausgangsprodukte der Verbrennungsreaktion 51 entstehen Kohlenmonoxid 51c, Lithiumoxid und Lithiumcarbonat 51d/e. Das Lithiumoxid und -Carbonat kann elektrochemisch reduziert werden und somit zu Lithium zurückgeführt werden. Dies geschieht z.B. über Zwischenschritte wie z.B. Chlorid. Das Kohlenmonoxid 51c stellt zugleich das Eingangsprodukt 52a für den folgenden Syntheseschritt dar. Es wird in den Synthesegasreaktor 52 eingeleitet. Dazu kommt als weiteres Eingangsprodukt Wasserstoff 52d. Mit dem Kohlenmonoxid 52a und dem Wasserstoff 52d zusammen erhält man somit Synthesegas, das unter anderem für die Methanolherstellung verwendet werden kann. Methanol 52c ist also eines der Ausgangsprodukte des Synthesegasreaktors 52 bzw. des Syntheseschrittes.

Ein Oxyfuel-Kraftwerk A ist eine moderne Anlage zur Erzeugung elektrischer Energie. Dabei fällt Kohlendioxid in hochkonzentrierter Form an. Des Weiteren fällt relativ reiner Stickstoff an. Werden diese Gase weiter genutzt, steigt die Effektivität des Gesamtsystems. Die Prozesskette wird durch die Reduktion von Kohlendioxid und Stickstoff verlängert. Über die Ausgangsprodukte wie z.B. Ammoniak kann eine Umstrukturierung des Düngemittelmarktes erfolgen. Das Ausgangsprodukt Methanol ist bedeutend für die Kraftstoffherstellung, da diese nun nicht mehr aus fossilen Rohstoffen als Energieträger, sondern umwelt- und klimafreundlich mittels des Sonnenenergiespeichers Lithium erzeugt werden. Lithium wird deshalb als Sonnenenergiespeicher bezeichnet, da es mittels photovoltaisch erzeugter elektrischer Energie aus seinen Oxiden oder Salzen gewonnen werden kann.

Somit kann der Markt fossiler Energieträger Volumen an den wachsenden Alkalimetall- bzw. Lithiummarkt abgeben. Zur Erzeugung der gleichen Menge thermischer Energie werden durch ein erfindungsgemäßes Kraftwerk weniger fossile Brennstoffe benötigt. Die Kohlenstoffatome, die für die Herstellung von Kraftstoffen benötigt werden, stammen aus Kohlendioxid niedriger Entropie, d.h. hochkonzentriertem Kohlendioxid. Bei der Verbrennung im Kraftfahrzeug wird es zu Kohlendioxid hoher Entropie und kann dann nur noch von Pflanzen zurückgeführt werden. Der Kraftstoffmarkt wird vergleichbar mit Kraftstoffen aus Biomasse kohlendioxidneutral. Der Alkalimetall- bzw. Lithiummarkt wird dadurch stark anwachsen. Zugleich werden sich Märkte in der Entwicklung einer Lithiuminfrastruktur entwickeln. Zum einen sind dies die Erzeugung von Lithium, zum anderen dessen Recycling.

**Patentansprüche**

1. Verfahren zur industriellen Verwertung der Stoffe Kohlenstoffdioxid und Stickstoff, bei dem die Stoffe in einem Verbrennungsschritt mit einem elektropositiven Metall umgesetzt werden und wenigstens ein Verbrennungsprodukt des Verbrennungsschritts in einem Reaktionsschritt weiter umgesetzt wird, bei dem in dem Verbrennungsschritt kraftwerkstechnisch nutzbare thermische Energie erzeugt und diese in elektrische Energie umgewandelt wird, wobei das Kohlendioxid aus einer Verbrennung eines Brennstoffs mit Sauerstoff stammt, und wobei der Stickstoff und der Sauerstoff aus der Luft abgetrennt wurden und wobei das elektropositive Metall ein Metall der ersten oder der zweiten Hauptgruppe oder ein Metall mit einem Normalpotential kleiner als null Volt ist.

2. Verfahren nach einem der vorstehenden Ansprüche, bei dem in dem Verbrennungsschritt Kohlendioxid mit dem elektropositiven Metall so umgesetzt wird, dass Kohlenmonoxid entsteht, und wobei in dem Reaktionsschritt eine Synthese durchgeführt wird, bei der aus dem Kohlenmonoxid durch Zugabe von Wasserstoff insbesondere Methanol hergestellt wird.

3. Verfahren nach einem der vorstehenden Ansprüche, bei dem in dem Verbrennungsschritt Stickstoff mit dem elektropositiven Metall so umgesetzt wird, dass ein Nitrid des elektropositiven Metalls entsteht, und wobei in dem Reaktionsschritt eine Hydrolyse durchgeführt wird, bei der aus dem Nitrid durch Zugabe von Wasser Ammoniak hergestellt wird oder bei dem der Verbrennungs- und der Reaktionsschritt zu einen Prozessschritt zusammengefasst sind und der Stickstoff und das Wasser mittels des elektropositiven Metalls direkt zu Ammoniak umgesetzt werden.

4. Verfahren nach einem der vorstehenden Ansprüche, bei dem vor dem Verbrennungsschritt ein Schritt zur Erzeugung elektrischer Energie durchgeführt wird, wobei in dem Schritt eine Verbrennung eines Brennstoffes mit Sauerstoff derart durchgeführt wird, dass dabei hochkonzentriertes Kohlendioxid anfällt, wobei das Kohlendioxid dem Verbrennungsschritt der Verwertung zugeführt wird.

5. Verfahren nach Anspruch 4, bei dem der Schritt zur Erzeugung elektrischer Energie ein Oxyfuel-Prozess ist.

6. Verfahren nach einem der vorstehenden Ansprüche, bei dem wenigstens ein Rückgewinnungsprozess durchgeführt wird, in dem ein Oxid und/oder ein Salz des elektropositiven Metalls in das Metall umgesetzt wird, wobei für den Rückgewinnungsprozess insbesondere photovoltaische Energie verwendet wird.

7. Vorrichtung (Z1/Z2) zur industriellen Verwertung der Stoffe Kohlenstoffdioxid und Stickstoff mit

   - jeweiligen Brennkammern (41/51), die für einen Verbrennungsschritt eines elektropositiven Metalls mit den Stoffen ausgestaltet sind, bei dem kraftwerkstechnisch nutzbare thermische Energie erzeugt wird, wobei das elektropositive Metall ein Metall der ersten oder der zweiten Hauptgruppe oder ein Metall mit einem Normalpotential kleiner als null Volt ist,
   - einer Reaktionskammer (42/52), die ausgestaltet ist ein Verbrennungsprodukt des Verbrennungsschritts in einem Reaktionsschritt weiter umzusetzen,
   - einem Kraftwerk (A) zur Erzeugung elektrischer Energie, wobei die Vorrichtung so ausgestaltet ist, dass die in dem Verbrennungsschritt erzeugte kraftwerkstechnisch nutzbare thermische Energie in elektrische Energie umgewandelt wird, und
   - einer Luftzerlegungsanlage (20) zur Abtrennung von Stickstoff und Sauerstoff aus der Luft, wobei die Luftzerlegungsanlage derart verbunden ist, dass der Stickstoff der jeweiligen Brennkammer (41) zur industriellen Verwertung als Stoff zuführbar ist, wobei das Kraftwerk (A) einen Dampferzeuger(31) zur Verbrennung eines Brennstoffes mit dem Sauerstoff umfasst, und wobei der Dampferzeuger derart verbunden ist, dass das bei der Verbrennung des Brennstoffes mit dem Sauerstoff entstehende Kohlendioxid (20a/40a) der jeweiligen anderen Brennkammer (51) zur Verwertung als Stoff zuführbar ist.

8. Vorrichtung (Z1/Z2) zur industriellen Verwertung eines Stoffes nach Anspruch 8 mit einer Wärmetransporteinrichtung (12), über die das Kraftwerk (A) mit den jeweiligen Brennkammern (41, 51) der Vorrichtung zur Verwertung verbunden ist, so dass thermische Energie, die durch den Verbrennungsschritt eines elektropositiven Metalls in der Brennkammer (41/51) erzeugt wird, transportiert und dem Kraftwerk (A) zusätzlich zur Verfügung gestellt werden kann.

9. Vorrichtung nach Anspruch 7 oder 8, wobei das Kraftwerk (a) ein Oxyfuel-Kraftwerk ist.

## Claims

1. Method for the industrial utilization of the materials carbon dioxide and nitrogen, in which the materials are reacted with an electropositive metal in a combustion step and at least one combustion product of the combustion step is reacted further in a reaction step, in which, in the combustion step, thermal energy capable of being used in power plants is generated and is converted into electrical energy, the carbon dioxide originating from a combustion of a fuel with oxygen, and the nitrogen and the oxygen having been separated from the air and the electropositive metal being a metal of the first or the second main group or a metal with a normal potential lower than zero Volt.

2. Method according to one of the preceding claims, in which, in the combustion step, carbon dioxide is reacted with the electropositive metal such that carbon monoxide occurs, in the reaction step a synthesis being carried out in which, in particular, methanol is produced from the carbon monoxide by the addition of hydrogen.

3. Method according to one of the preceding claims, in which, in the combustion step, nitrogen is reacted with the electropositive metal such that a nitride of the electropositive metal occurs, in the reaction step hydrolysis being carried out in which ammonia is produced from the nitride by the addition of water, or in which the combustion step and reaction step are combined into one process step and the nitrogen and water are converted directly into ammonia by means of the electropositive metal.

4. Method according to one of the preceding claims, in which, before the combustion step, a step for the generation of electrical energy is carried out, in the step combustion of a fuel with oxygen being carried out in such a way that highly concentrated carbon dioxide in this case occurs, the carbon dioxide being delivered to the combustion step for utilization.

5. Method according to Claim 4, in which the step for the generation of electrical energy is an oxyfuel process.

6. Method according to one of the preceding claims, in which at least one recovery process is carried out, in which an oxide and/or a salt of the electropositive metal are/is converted into the metal, in particular photovoltaic energy being used for the recovery process.

7. Apparatus (Z1/Z2) for the industrial utilization of the materials carbon dioxide and nitrogen, with

   - respective combustion chambers (41/51) which are configured for a combustion step of an electropositive metal with the materials, in which thermal energy capable of being used in power plants is generated, the electropositive metal being a metal of the first or the second main group or a metal with a normal potential lower than zero Volt,
   - a reaction chamber (42/52) which is configured to react further a combustion product of the combustion step in a reaction step,
   - a power plant (A) for the generation of electrical energy, the apparatus being configured such that the thermal energy generated in the combustion step and capable of being used in power plants is converted into electrical energy, and
   - an air separation plant (20) for the separation of nitrogen and oxygen from the air, the air separation plant being connected such that the nitrogen is capable of being delivered as a material to the respective combustion chamber (41) for industrial utilization, the power plant (A) comprising a steam generator (31) for the combustion of a fuel with oxygen, and the steam generator being connected such that the carbon dioxide (20a/40a) which occurs during the combustion of the fuel with oxygen is capable of being delivered as a material to the respective other combustion chamber (51) for industrial utilization.

8. Apparatus (Z1/Z2) for the industrial utilization of a material according to Claim 8, with a heat transport device (12), via which the power plant (A) is connected to the respective combustion chambers (41, 51) of the apparatus for utilization, so that thermal energy which is generated by means of the combustion step of an electropositive metal in the combustion chamber (41/51) can be transported and additionally made available to the power plant (A).

9. Apparatus according to Claim 7 or 8, the power plant (a) being an oxyfuel power plant

**Revendications**

1. Procédé de valorisation industrielle des substances dioxyde de carbone et azote, dans lequel on fait réagir les substances, dans un stade de combustion, sur un métal électropositif et on continue à faire réagir, dans un stade de réaction, au moins un produit de combustion du stade de combustion, dans lequel on produit, dans le stade de combustion, de l'énergie thermique utilisable en technique de centrale électrique et on la transforme en énergie électrique, dans lequel le dioxyde de carbone provient d'une combustion d'un combustible par de l'oxygène et dans lequel l'azote et l'oxygène ont été séparés de l'air et dans lequel le métal électropositif est un métal du premier ou du deuxième groupe principal ou un métal ayant un potentiel normal plus petit que zéro volt.

2. Procédé suivant l'une des revendications précédentes, dans lequel on fait réagir, dans le stade de combustion, du dioxyde de carbone sur le métal électropositif, de manière à produire du monoxyde de carbone et dans lequel on effectue, dans le stade de réaction, une synthèse dans laquelle on produit, à partir du monoxyde de carbone, par addition d'hydrogène, notamment du méthanol.

3. Procédé suivant l'une des revendications précédentes, dans lequel on fait réagir, dans le stade de combustion, de l'azote sur le métal électropositif, de manière à produire un nitrure du métal électropositif et dans lequel on effectue, dans le stade de réaction, une hydrolyse dans laquelle on produit de l'ammoniac à partir du nitrure, par addition d'eau, ou dans lequel on rassemble le stade de combustion et de réaction en un stade opératoire et on transforme, directement en ammoniac, l'azote et l'eau au moyen du métal électropositif.

4. Procédé suivant l'une des revendications précédentes, dans lequel on effectue, avant le stade de combustion, un stade de production d'énergie électrique, dans lequel, dans le stade, on effectue une combustion d'un combustible par de l'oxygène, de manière à produire du dioxyde de carbone très concentré, le dioxyde de carbone étant, pour la valorisation, envoyé au stade de combustion.

5. Procédé suivant la revendication 4, dans lequel le stade de production d'énergie électrique est un processus oxyfuel.

6. Procédé suivant l'une des revendications précédentes, dans lequel on effectue au moins une opération de récupération, dans laquelle on transforme un oxyde et/ou un sel du métal électropositif en le métal, de l'énergie, notamment photovoltaïque, étant utilisée pour l'opération de récupération.

7. Installation (Z1, Z2) de valorisation industrielle des substances dioxyde de carbone et azote, comprenant

   - des chambres de combustion (41/51) respectives, constituées pour un stade de combustion d'un métal électropositif par les substances, dans lequel on produit de l'énergie thermique utilisable en technique de centrale électrique, le métal électropositif étant un métal du premier ou du deuxième groupe principal, ou un métal ayant un potentiel normal plus petit que zéro volt
   - une chambre (42, 52) de réaction, constituée pour faire réagir davantage, dans un stade de réaction, un produit de combustion du stade de combustion,
   - une centrale (A) de production d'énergie électrique, l'installation étant conformée de manière à transformer en énergie électrique l'énergie thermique utilisable en technique de centrale électrique produite dans le stade de combustion et
   - une installation (20) de fractionnement de l'air pour séparer de l'azote et de l'oxygène de l'air, l'installation de fractionnement de l'air communiquant de manière à pouvoir, comme substance pour la valorisation industrielle, envoyer l'azote à la chambre de combustion (41) respective,
   dans laquelle la centrale (A) comprend un générateur (31) de vapeur pour la combustion d'un combustible par de l'oxygène et dans laquelle le générateur de vapeur communique de manière à pouvoir envoyer, comme substance pour la valorisation, du dioxyde de carbone (20a/40a), produit par la combustion du combustible par l'oxygène, à respectivement l'autre chambre de combustion (51).

8. Installation (Z1/Z2) de valorisation industrielle d'une substance suivant la revendication 8, comprenant un dispositif (12) de transport de chaleur, par lequel la centrale (A) est, pour la valorisation, reliée aux autres chambres de combustion (41, 51) de l'installation, de manière à pouvoir transporter de l'énergie thermique produite par le stade de combustion d'un métal électropositif dans la chambre de combustion (41/51) et à pouvoir le mettre à disposition supplémentairement de la centrale (A).

9. Installation suivant la revendication 7 ou 8, dans laquelle la centrale (a) est une centrale oxyfuel.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102009014026 A1